(19) Europäisches Patentamt
European Patent Office
Office européen des brevets

(11) **EP 2 870 473 B1**

(12) **EUROPEAN PATENT SPECIFICATION**

(45) Date of publication and mention
of the grant of the patent:
**01.02.2017 Bulletin 2017/05**

(51) Int Cl.:
**C02F 5/10** (2006.01)      **C02F 1/42** (2006.01)
**G01N 33/18** (2006.01)

(21) Application number: **13734075.8**

(22) Date of filing: **04.07.2013**

(86) International application number:
**PCT/EP2013/064112**

(87) International publication number:
**WO 2014/006129 (09.01.2014 Gazette 2014/02)**

(54) **DETERMINATION OF A CONVERSION FACTOR RELATING THE CONDUCTIVITY AND THE HARDNESS OF WATER**

**BESTIMMUNG EINES UMRECHNUNGSFAKTORS ZWISCHEN DER LEITFÄHIGKEIT UND DER HÄRTE VON WASSER**

**DÉTERMINATION D'UN FACTEUR DE CONVERSION ENTRE LA CONDUCTIVITÉ ET LA DURETÉ DE L'EAU**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2012 EP 12175156**

(43) Date of publication of application:
**13.05.2015 Bulletin 2015/20**

(73) Proprietor: **Brita GmbH**
**65232 Taunusstein (DE)**

(72) Inventors:
• **WEIDNER, Peter**
**92444 Rötz (DE)**
• **CONRADT, Berthold**
**65205 Wiesbaden (DE)**
• **NAGEL, Thomas**
**35796 Weinbach (DE)**

(74) Representative: **van Lookeren Campagne, Constantijn August**
**Meissner Bolte Patentanwälte**
**Rechtsanwälte Partnerschaft mbB**
**Bankgasse 3**
**90402 Nürnberg (DE)**

(56) References cited:
**WO-A2-2009/071066      DE-A1-102010 001 373**

**Description**

The invention relates to a method of determining a measure of a concentration of components removable from fluid in the form of a liquid by a fluid treatment part of a fluid treatment device, including the use of a fluid treatment apparatus including:

an inlet for receiving untreated fluid;
an outlet; and
at least one fluid path between the inlet and the outlet including at least one fluid treatment part in the form of a liquid treatment part for treating fluid to remove to at least a certain extent at least some types of components in fluid led through the fluid treatment part, the method including:

obtaining at least two measurement values, each representative of a respective value of a parameter of the fluid depending partly on the concentration of components removable by the fluid treatment part,
wherein the measurement values pertain to measurements carried out on fluid at different ratios of untreated fluid to fluid led through the at least one fluid path including a fluid treatment part;
using the measurement values to obtain a difference value representative of a change in the value of the parameter due to treatment by the fluid treatment part; and
converting the difference value into an output value representative of the concentration of components removable from fluid by a fluid treatment part.

The invention also relates to a system for determining a measure of a concentration of components removable from fluid in the form of a liquid by a fluid treatment part of a fluid treatment device based on measurements obtained using a fluid treatment apparatus including:

an inlet for receiving untreated fluid;
an outlet; and
at least one fluid path between the inlet and the outlet including at least one fluid treatment part in the form of a liquid treatment part for treating fluid to remove to at least a certain extent at least some types of components in fluid led through the fluid treatment part, the system including:

an interface to at least one sensor for obtaining at least two measurement values, each representative of a respective value of a parameter of the fluid depending partly on the concentration of components removable by the fluid treatment part,
wherein the measurement values pertain to measurements carried out on fluid at different ratios of untreated fluid to fluid led through the fluid path including the fluid treatment part,
wherein the system is configured to use the measurement values to obtain a difference value representative of a change in the value of the parameter due to treatment by the fluid treatment part; and
to convert the difference value into an output value representative of the concentration of components removable from fluid by a fluid treatment part.

The invention also relates to a system for treating a fluid.
The invention also relates to a computer program.
DE 10 2010 001 373 A1 discloses a method of operating a water treatment apparatus with a softening device, in particular one comprising an ion exchange resin, and an automatically adjustable blending device for mixing a diluent water flow from a first, softened partial flow and a second, raw water-carrying water flow. The method includes a step of determining the electrical conductivity of the raw, softened or blended water by means of a conductivity sensor and, based thereon, determining the total hardness of the raw water through one or more characteristic calibration curves stored in an electronic control device. In an embodiment, the characteristic calibration curve is a straight line through the origin that can be completely described by its slope. The method includes controlling the adjustment position of the blending device by means of the electronic control device, taking account of the determined total hardness of the raw water, so that the hardness in the blended water flow is set to a prescribed target value. The hardness of the blended water flow is determined in a non-conductometric manner and compared with the target value. One or more characteristic calibration curves for determining the total hardness of the raw water are modified, if a deviation of the non-conductometrically determined blended water hardness from the target value exceeds a threshold value.
EP 2 169 393 A1 discloses a method of determining the hardness of water. A first electrical conductivity value $\sigma_{CaH2O}$ is determined by means of a first measurement carried out on untreated water, and a second electrical conductivity value $\sigma_{NaH2O}$ is determined by means of a measurement carried out on treated water. The untreated water is treated by an ion exchanger to obtain softened, treated water. The second measurement is effected on the softened, treated water. The ion exchanger exchanges calcium ions against sodium ions, wherein the electrical conductivity of the water is changed. This change can be determined with the second measurement. A concentration $c_{Ca}$ of calcium ions of the untreated water is determined using the equation:

$$c_{Ca} = \left(\sigma_{NaH2O} - \sigma_{CaH2O}\right) \Big/ \left(2 * \Lambda o_{Na} - \Lambda o_{Ca}\right),$$

wherein $\Lambda o_{Ca}$ is the molar conductivity of calcium at 25°C and $\Lambda o_{Na}$ is the molar conductivity of sodium at 25°C. If the contribution of magnesium ions to the hardness of the water is negligible, then the hardness of the water can be derived from the concentration $c_{Ca}$ of the calcium ions without further ado. For example, the hardness of the water can be indicated in units of French hardness using the known relation $1°fH = 0.2 * c_{Ca}$.

A problem of this determination is that it is insufficiently accurate for all types of mains water. In some types of mains water, there are many additional ion species. Although their contribution to the measured conductivity is removed by taking the difference between the value before and after the ion exchanger, the conversion into a hardness value is not sufficiently accurate for all purposes.

It is an object of the invention to provide a method, systems and computer program that yield a relatively accurate measure of a concentration of components removable from fluid by a fluid treatment part of a fluid treatment device, such as a measure of temporary hardness.

This object is achieved according to a first aspect by the method according to the invention, which is characterised in that the conversion is carried out in dependence on a further value of the parameter approximating absolute values of the parameter within the fluid treatment apparatus.

The method makes use of at least two measurement values, each representative of a respective value of a parameter of the fluid depending partly on the concentration of components removable by the treatment part. An example is the electrical conductivity, which depends on the concentration of all ion species, not just those removable by a particular fluid treatment device. The method therefore does not rely on an (expensive) ion-selective sensor (even if such a sensor would be available). Because the measurement values pertain to measurements carried out on fluid at different ratios of untreated fluid to fluid led through the fluid path including the fluid treatment part, it is possible to use the measurement values to obtain a difference value, representative of a change in the value of the parameter due to treatment by the fluid treatment part. This difference captures the effect due to the fluid treatment and thus the concentration of removable components present in the untreated fluid. The difference value is converted into an output value representative of the concentration of components removable from fluid by a fluid treatment part. The method is based on the realisation that accuracy can be improved by carrying out the conversion in dependence on a value of the parameter at least approximating the absolute values of the parameter of the fluid within the fluid treatment apparatus. This takes account of the fact that properties such as the conductivity or pH of a fluid like water do not vary exactly linearly with e.g. the ion concentration, but are dependent on activity coefficients that are concentration-dependent.

In an embodiment, the further value at least approximates absolute values of the parameter directly upstream and directly downstream of the fluid treatment part.

This further improves the accuracy, since it is the difference between these two absolute values that is to be converted into the output value. Because the difference value equals the difference between two parameter values, a value that approximates at least one of them, but may be equal to the other of them, will need to be used in the conversion.

In a variant, the further value corresponds to an average of the values of the parameter directly upstream and downstream of the fluid treatment part.

Thus, in this case, the further value approximates both the parameter value directly upstream and directly downstream of the fluid treatment part, being a value in between.

In an embodiment, converting the difference value into an output value includes dividing the difference value by a factor linearly dependent on the further value.

It is known that the activity of a dissolved substance, in particular of a particular ion species, is dependent on its concentration and that of other ion species (this follows from the Debye-Hückel-Onsager theory). To a first approximation, the dependency is a linear one, with the conversion factor decreasing in proportion to the parameter value. This has been confirmed experimentally for an application in which the output value to be determined is the temporary hardness of water and the parameter is the electrical conductivity, using a relatively large number of water samples supplied by different utilities. Hence, this variant gives relatively accurate results whilst keeping the computational resources required to obtain the output value within acceptable limits.

In an embodiment, the measurement values are based on measurements of the electrical conductivity of the fluid.

The electrical conductivity is relatively easily measured and processed. It depends on the concentration of dissolved salts, which are commonly removed or replaced by fluid treatment parts of fluid treatment devices.

In an embodiment, the measurement values are further dependent on at least one measurement of temperature.

Thus, a further source of inaccuracy is removed. It is only necessary to store one set of constants for the conversion of the difference value into the output value, namely a set that is valid for a reference temperature. In one implementation, the measurement values are obtained from a sensor device including a temperature sensor and an electrical conductivity sensor, which sensor device is configured to carry out a correction of measured conductivity values taking account of deviations of the temperature from a reference temperature to provide corrected measurement values as output. In another variant, a sensor device incorporating a temperature-dependent electrical component, for example a temperature-dependent resistor, is used to obtain a measurement value dependent on both temperature and the value of the parameter of the fluid. Both types of sensor device are commonly obtainable and place only limited requirements on the computational resources of the data processing device that calculates the output value.

In an embodiment, the measurement values are obtained from the same sensor at different points in time.

This embodiment avoids the problem of sensor drift that would occur if the measurement values used to obtain the difference value were to originate from different sensors. Subtracting the two values would not remove the influence of sensor drift, so that periodical re-calibration would be required. Variations in the composition of untreated fluid such as mains water on a short time scale are generally small, so that using successive measurements does not introduce a relevant error. Moreover, the use of one sensor is more cost-effective.

In a variant of this embodiment, the fluid treatment apparatus further includes:

a branch point between the inlet, the at least one fluid path including at least one fluid treatment part and at least one second fluid path, each second fluid path bypassing at least one of the fluid treatment parts, the second fluid paths conducting, in use, a blending fraction between zero and one of the fluid received through the inlet;

a mixing location, where the first and second fluid paths join; and

at least one device for adjusting the blending fraction,

wherein the method includes obtaining the measurement values from at least one sensor located downstream of the mixing location and causing the at least one device to adjust the blending fraction between the measurements carried out at different ratios of untreated fluid to fluid led through the at least one fluid path including at least one fluid treatment part.

The fluid at the mixing location is a mix of fluid treated by the fluid treatment parts and fluid that has bypassed these fluid treatment parts. Thus, treated fluid is diluted by blending it with untreated fluid. The blending fraction is a measure of how much of the total untreated fluid is left untreated and then mixed with the treated fluid. Fluid treatment apparatus as defined above may in particular include a fluid treatment part configured to remove temporary or carbonate hardness from liquids. Such a fluid treatment part will include an ion exchange medium that not only removes components contributing to temporary hardness, but in the process lowers the pH of the liquid. By blending liquid that has been treated with liquid that has been left untreated, the resultant mix is not too acid. This protects appliances provided with the mix from corrosion.

From the known blending fractions at which the measurements are carried out, the measurement values are converted into a difference value representative of the change in value of the parameter due to treatment by the fluid treatment part. This can be done, for example, by dividing the change in measurement value by the change in blending fraction. This variant of the method allows the use of one single sensor. Because it in particular avoids the use of a sensor located upstream of the fluid treatment part, impairment of the sensor due to components removable by the fluid treatment part (e.g. impairments due to scale deposition) are reduced. The method can use quite small changes in the blending fraction. These can therefore be made around a value determined to be optimal for an appliance to which the mix of fluid downstream of the mixing location is provided. Thus, for example, where the fluid treatment part is arranged to remove temporary hardness from liquids, the appliance provided with a mix of treated and untreated liquid is not provided with (corrosive) fully treated water.

A further effect is to make the method suitable for use with a common type of existing fluid treatment apparatus. This type of apparatus includes a filter head and a replaceable filter cartridge. The untreated fluid is split in the filter head into two flows that are led into the cartridge. One flow of fluid is directed through a filter bed, e.g. for softening. The other flow bypasses the filter bed. The two flows are joined in the cartridge and the joined flow may undergo a further treatment (e.g. to remove volatile organic compounds or bacteria). The cartridge has a single outlet for delivering the treated fluid back to the filter head. Carrying out measurements on only the fluid that has passed through the filter bed would mean placing a sensor in the cartridge, which is generally not a feasible proposition. By obtaining the measurement values from at least one sensor located downstream of the mixing location, it is possible to use a sensor placed in the filter head or even downstream of the filter head.

The device for adjusting the blending fraction will generally include a motor, for example a stepper motor or servomotor, coupled to one or more valves. It may further include a torque-converting gear unit.

In an embodiment of the method, the fluid treatment part is a liquid treatment part including an ion exchange medium, more particularly a liquid treatment part including an ion exchange medium that is at least initially in a hydrogen form.

This embodiment results in relatively large difference values, because the fluid treatment part is configured to remove cations from the liquid. Cations such as calcium and magnesium are exchanged for hydrogen ions, which react with carbonate and hydrogen carbonate anions to form water and carbon dioxide. Thus, the concentration of charge carriers is reduced. This is reflected in a relatively large difference value, especially where the measurement values are values representative of electrical conductivity of the liquid (optionally normalised to a reference temperature). In the alternative, the change in parameter value would be smaller, because cations would be exchanged for cations of the same valence. In that case, there would still be changes in e.g. electrical conductivity due to the fluid treatment, but they would be down to different activity levels for the different types of ions, and therefore smaller. The ion exchange medium in the hydrogen form may be the only type of cation exchange medium included in fluid treatment parts in the fluid path. The ion exchange medium may in particular be a weakly acidic ion exchange medium.

An embodiment of the method includes:

obtaining a measure of an amount of fluid led through at least one fluid path including at least one fluid treatment part; and
using at least this measure and a measure of the concentration of components removable from fluid determined for the certain time period in a determination of a measure of a state of exhaustion of the fluid treatment part.

In this embodiment, an integral of the flow rate, weighted according to the concentration of components removable by the fluid treatment par, is determined. This represents the accumulated load on the fluid treatment part during its lifetime. The method allows an automated system carrying it out to determine the state of exhaustion, or conversely the remaining useful lifetime (also a measure of the state of exhaustion), of the fluid treatment part and/or the fluid treatment medium it contains. Where the fluid treatment part is comprised in a replaceable cartridge, the system carrying out this embodiment is able to determine when the cartridge should be replaced. In one variant, an output representative of the state of exhaustion is caused to be provided by an output device in a form perceptible to a user. In other words, an audible or visible signal is provided to the user. Additionally or alternatively, output date representative of the state of exhaustion are communicated to an external device, for example an appliance arranged to receive the treated fluid, via a communication link. This would allow the user interface of the external device to be used to provide a signal to the user. It is useful where the fluid treatment apparatus is located out of sight of the user, but the appliance is not. In a variant, the output perceptible to a user is only provided upon determining that fluid is being drawn from the fluid treatment apparatus. This increases the likelihood that a user is actually present to observe the output, and saves energy. Similarly, the communication of output data to an external device via a communication link can be effected in dependence upon a determination that fluid is being drawn from the fluid treatment apparatus. This increases the likelihood that the data will be processed, since the external device is unlikely to be switched off or in a standby mode.

According to another aspect, the system for determining a measure of a concentration of components removable from fluid by a fluid treatment part of a fluid treatment device is characterised in that the system is configured to carry out the conversion in dependence on a further value of the parameter approximating absolute values of the parameter within the fluid treatment apparatus.

The system provides essentially the same effects as the method. It is suitable as only a measurement system to provide the output value in the form of output through an interface to a data processing system or user interface. It can also be part of a control system for controlling the concentration of components removable by the fluid treatment part in the fluid provided at the output, e.g. by varying the fraction of fluid provided at the inlet that bypasses the fluid treatment part.

In an embodiment, the system is configured to carry out a method according to the invention.

According to another aspect, the system for treating a fluid according to the invention includes at least:

an inlet for receiving untreated fluid;
an outlet;
at least one fluid path between the inlet and the outlet including a fluid treatment part for treating fluid to remove to at least a certain extent at least some types of components in fluid led through the fluid treatment part; and
a system for determining a measure of a concentration of components removable from fluid by a fluid treatment part of a fluid treatment device according to the invention.

According to another aspect of the invention, there is provided a computer program including a set of instructions capable, when incorporated into a machine-readable medium, of causing a system having information processing capabilities to carry out a method according to the invention.

The invention will be explained in further detail with reference to the accompanying drawings, in which:

Fig. 1 is a schematic diagram of a water treatment apparatus including a system for determining a measure of temporary hardness;

Fig. 2 is a flow chart illustrating steps in a method used by the system to determine a measure of temporary hardness;

Fig. 3 is a diagram showing the fit of a variable conversion factor used in the method of Fig. 2 to experimental data;

Fig. 4 is a schematic diagram of an alternative water treatment apparatus for use in a variant of the method of Fig. 2; and

Fig. 5 is a schematic diagram of another alternative water treatment apparatus for use in a variant of the method of Fig. 2.

In the following, the example of a liquid treatment system for softening water will be used. The apparatus is equally suitable for treating other types of liquid, and the methods discussed below for determining a measure of hardness can also be used to determine the concentration of components removable by a liquid treatment part of a liquid treatment device for treating such other liquids.

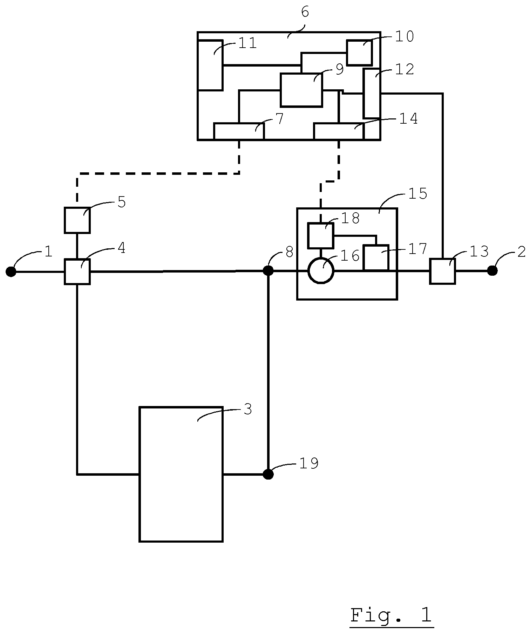

A water treatment apparatus illustrated by way of example in Fig. 1 includes an inlet 1 for receiving untreated water and an outlet 2 for providing water with a desired level of temporary hardness. The inlet 1 is connectable to a supply of untreated water, in particular the mains water supply. The outlet 2 is connectable to an appliance such as a steam cooker, dish washer or a device for preparing beverages (e.g. a coffee maker). The fluid treatment apparatus includes a fluid treatment device 3 configured to remove temporary hardness to at least a certain extent from the water led through it. In the following, it will be assumed for simplicity that the fluid treatment device 3 is effective to remove essentially all temporary hardness. Temporary hardness, also known as carbonate hardness, is caused by the presence of dissolved carbonate minerals (essentially carbonate and magnesium carbonate). It is distinguished from permanent hardness, to which other minerals such as calcium chloride also contribute.

In an alternative embodiment, the fluid treatment device 3 is effective to remove not just the temporary hardness but all hardness from the water led through it, and the fluid treatment apparatus is configured to supply water with a desired level of total hardness. This is merely a question of using a fluid treatment device 3 with a different filter medium. Returning to the example of temporary hardness, the fluid treatment device 3 can comprise a filter medium including a weakly acidic ion exchanger that is at least initially in the hydrogen form, for example.

There is thus a first fluid path running from the inlet 1 through the fluid treatment device 3 to the outlet. In the fluid treatment apparatus of Fig. 1, there is also a second fluid path from the inlet 1 to the outlet 2. This second fluid path bypasses the fluid treatment device 3. In the illustrated embodiment, the water passing through the second fluid path is not treated at all. In an alternative embodiment, it bypasses any fluid treatment parts that are of the same type as that of the first fluid path, but passes through one or more alternative fluid treatment parts. These may be configured to remove the same components, but to a different extent. In general, however, the alternative fluid treatment part will be arranged to remove different components and/or add components. As an example, the second fluid path may include a sediment filter or an activated carbon filter. In one embodiment, the fluid treatment part included in the first fluid path is included in a replaceable fluid treatment device. The second fluid path may pass through the same fluid treatment device, but bypass the fluid treatment part configured to remove temporary hardness.

A variable ratio flow divider 4 is arranged at a branch point where the first and second fluid paths go their separate ways. The flow divider 4 is adjustable by a motor 5 controlled by a control device 6 provided with an interface 7 to the motor 5. In this way, an adjustable fraction of the water passing through the inlet 1, which fraction is referred to herein as a blending fraction x, is led through the second fluid path. The first and second fluid paths join at a mixing location 8, so that the water treated by the fluid treatment device 3 and that left untreated can mix. In the illustrated embodiment, the control device 6 is programmed to relate positions of the motor 5 to values of the blending fraction x and vice versa.

The control device 6 includes a data processing unit 9 and memory 10. It is provided with an interface 11 for providing input relating to a target value of the temporary hardness of the water at the outlet 2. The input may be a value or information representative of the type of application the water is to be used in. In an alternative embodiment, such input can be communicated from another device through the interface 11. The control device 6 is configured to determine the temporary hardness of the water at the inlet and to set the blending fraction x to the appropriate value by adjusting the settings of the motor 5 and flow divider 4. In this way, a particular target value of the temporary hardness can be achieved even though the fluid treatment device 3 is able to remove the temporary hardness from water led through it to only a fixed extent, generally 100 %.

In the illustrated embodiment, the control device 6 includes an interface 12 to a flow meter 13 configured to measure the volumetric flow through the fluid treatment apparatus. Since the control device 6 controls the settings determining the blending fraction x, it can convert the total accumulated volumetric flow through the fluid treatment apparatus into a value representative of the volume of water that has passed through the fluid treatment device 3. Since it also determines the temporary hardness of the water at the inlet 1, it is able to determine a measure of the total amount of temporary hardness-inducing components to which the fluid treatment device 3 has been exposed over a certain period (generally commencing at first use). In effect, it calculates an integral over time of the flow rate, weighted by a measure of the hardness of the water passing through at that flow rate. In this way, it can signal when the fluid treatment device 3 has become exhausted and is to be replaced or regenerated. This signal is provided through the interface 11 or a separate output interface (not shown). Where the interface 11 is a user interface, the signal is provided in a form perceptible by a user, e.g. by way of a visible and/or audible indication of the remaining lifetime of the fluid treatment device 3 and/or of the fraction of initially available treatment capacity that has been used up. Where the interface 11 is a data communication interface for establishing a data communication link to an external appliance, the same information is communicated in the form of a data signal for processing and/or output in a perceptible form by the external appliance. Optionally, the signal is provided only upon determining that fluid is being provided through the outlet 2.

The control device 6 is also provided with an interface 14 to a sensor device 15 including an electrical conductivity sensor 16 arranged to measure the electrical conductivity of the water downstream of the mixing location 8. The electrical conductivity of water is dependent on the concentration of dissolved ions of all species, not just those contributing to hardness or that fraction of those species contributing to hardness that contributes to the temporary hardness. Thus, for example, the water can contain dissolved calcium chloride, of which the calcium ions do not contribute to temporary hardness, but do contribute to permanent hardness. Moreover, certain ion species do not contribute to hardness at all, but their concentration partly determines the electrical conductivity of the water. The control device 6, specifically the data processing unit 9, is programmed to determine the temporary hardness of the untreated water using measurement values obtained from the sensor device 15.

In the illustrated embodiment, the sensor device 15 includes a temperature sensor 17 and a data processor 18 for converting electrical conductivity values from the conductivity sensor 16 into values that would have been obtained if the temperature had been at a certain reference value, e.g. 25°C. These corrected values are provided as output to the control device 6. This takes account of the fact that the electrical conductivity for a given concentration varies with the temperature of the water. The temperature signal need not be provided to the control device 6, saving on connectors and leads and reducing the potential for failures.

The electrical conductivity of the water at the location of the sensor device 15, i.e. downstream of the mixing location 8 is dependent on the electrical conductivity of the untreated water, the blending fraction and the electrical conductivity of the water at an exit point 19 directly downstream of the fluid treatment device 3, but upstream of the mixing location 8. Let the electrical conductivity of the untreated water be $s_0$ and the electrical conductivity at the exit point 19 be $s_1$. The difference $\Delta s \equiv s_0 - s_1$ is due to the removal of the temporary hardness, i.e. representative of a change in the electrical conductivity due to treatment by the fluid treatment device 3. This value is to be obtained and converted into a measure of temporary hardness. The electrical conductivity of the water at the location of the sensor device 15 can be written as follows:

$$s = x \cdot s_0 + (1-x) \cdot s_1 = x \cdot (s_0 - s_1) + s_1 = x \cdot \Delta s + s_1 \ . \qquad (1)$$

It will be appreciated that the difference value $\Delta s$ can be obtained by determining a value representative of the derivative of the electrical conductivity $s$ at the location of the sensor device 15 with respect to the blending fraction x. This is essentially what the control device 6 is configured to do.

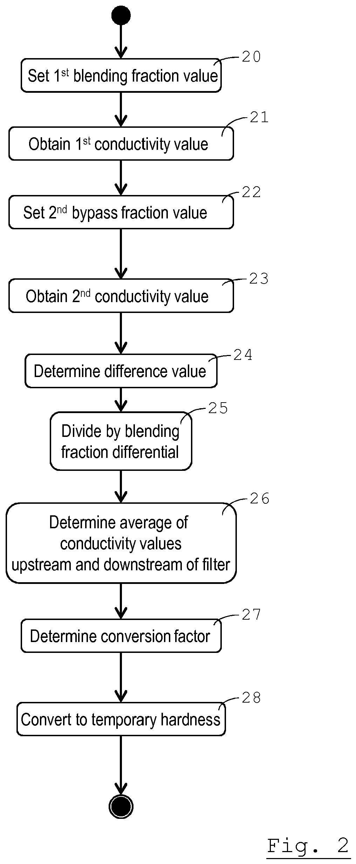

A method of obtaining one value of the temporary hardness of the untreated water is illustrated in Fig. 2. This method will be executed at intervals so as to capture common variations in the composition of the water supplied to the inlet 1. For example, the method may be executed daily or at intervals longer than a day.

In a first step 20, the control device 6 causes the motor 5 and flow divider 4 to adjust such that the blending fraction is changed to a first value $x_1$, e.g. $x_1 = x_0 + \Delta x/2$. In a next step 21, a measurement value $s(x_1)$ is obtained from the sensor device 15, the value pertaining to a measurement carried out with the blending fraction at the first value $x_1$. Next (step 22), the control device 6 causes the motor 5 and flow divider 4 to adjust such that the blending fraction is changed to a second value $x_2$, e.g. $x_2 = x_0 - \Delta x/2$. Then (step 23), a second measurement value $s(x_2)$ is obtained from the sensor device 15, the value pertaining to a measurement carried out with the blending fraction at the second value $x_2$. This step 23 is executed after a delay that is long enough to ensure that the sensor device 15 measures the electrical conductivity $s(x_2)$ at the new value $x_2$ of the blending fraction.

The control device can now determine (step 24) the difference value $s(x_2) - s(x_1)$ and (step 25) divide this value by the blending fraction differential $\Delta x$ to obtain an approximation of the derivative of the conductivity $s(x)$ with respect to the blending fraction x, which, it will be recalled, approximates the difference in conductivity caused by the temporary hardness that is removable by the fluid treatment device 3.

It would be possible to convert this value into a hardness value by dividing by a constant conversion factor F, e.g. $F = 30 \ \mu S/°dH$, where dH stands for deutsche Härte. However, this can yield insufficiently accurate results. Therefore, a variable factor F is used. This factor F varies linearly with the average of the conductivity $s_0$ directly upstream of the fluid treatment device 3 and the conductivity $s_1$ at the exit point 19, i.e. directly downstream of the fluid treatment device 3. Thus, in a next step 26, this average $\bar{s}$ is determined. One way of doing this is by calculating the average $\bar{s}$ using the following equation:

$$\bar{s} = s(x_1) + \tfrac{1}{2} \cdot \Delta s \; . \qquad\qquad (2)$$

A linear function of the type $F=-a \cdot \bar{s}+b$, where a and b are constants, stored in memory 10 is used to obtain the conversion factor (step 27). The temporary hardness follows (step 28) by dividing the result of the step 25 with which the difference in conductivity before and after the fluid treatment device 3 was determined by the value of the factor F obtained in the preceding step 27.

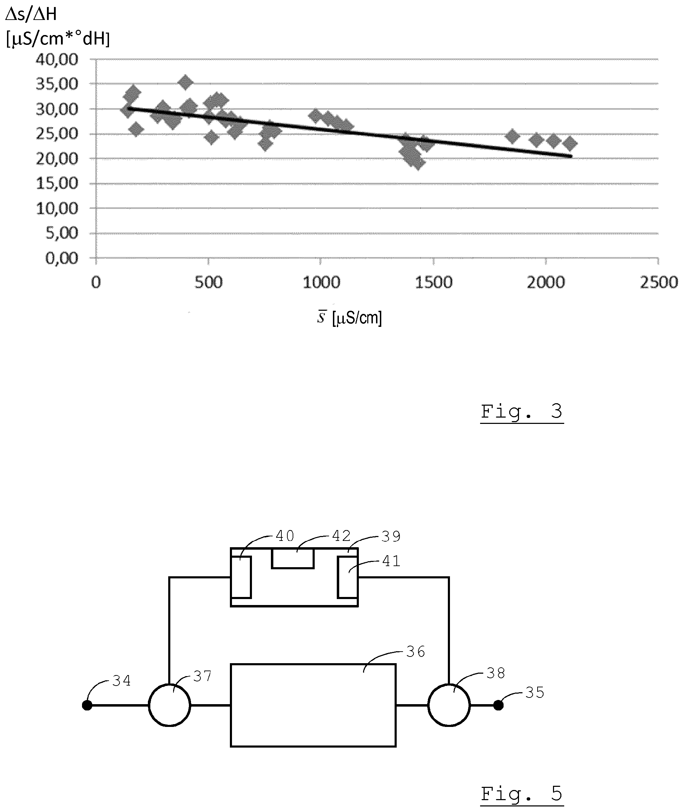

Fig. 3 is a diagram showing a graph that is a fit of the linear relation used to calculate the conversion factor F to data obtained using a multitude of water samples and data sets calculated using the Debye-Hückel-Onsager method. In fact, this is how suitable values of the constants a and b can be determined. The experimental data were obtained by passing water samples through a filter bed arranged to remove all temporary hardness. The electrical conductivity before and after the filter bed were determined. Additionally, the carbonate hardness was determined using titrimetry. The theoretical data sets were calculated using known compositions of water (i.e. a mixture of ions at respective concentrations), for which both the hardness levels were calculated (based on the concentration of $Mg^{2+}$ and $Ca^{2+}$ ions) and the theoretically expected electrical conductivity were calculated (using the Debye-Hückel-Onsager theory).

It will be appreciated from the diagram of Fig. 3, which as mentioned reflects experimental data, that the linear relation is a relatively good fit and at least an improvement over a constant conversion factor.

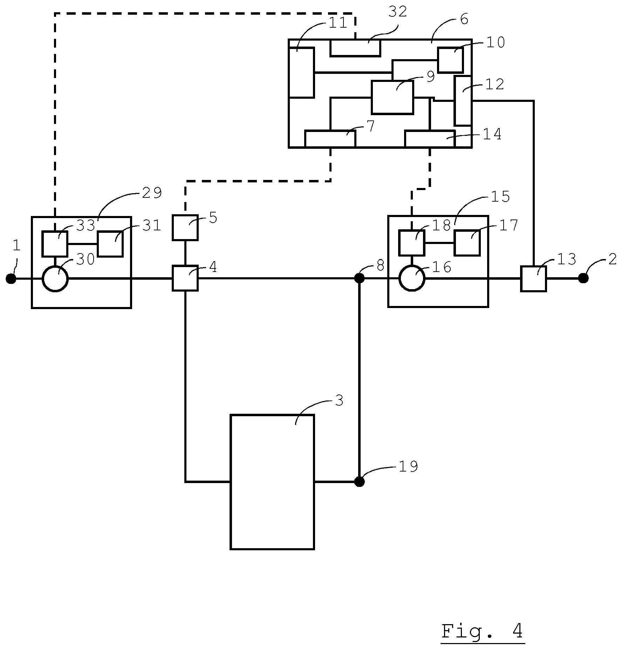

Fig. 4 is a schematic diagram of an alternative liquid treatment system in which like parts have been given like reference numerals. The alternative system differs in that it includes a second sensor device 29. It includes a second electrical conductivity sensor 30 and a second temperature sensor 31, and is arranged to provide measurement values to the control device 6, which is provided with an appropriate further interface 32. The second sensor device 29 includes a data processor 33 adjusting values of the electrical conductivity obtained from the second electrical conductivity sensor 30 to take account of deviations from a reference temperature.

This variant of the system does not require the control device to vary the blending fraction x to obtain measurement values. Instead, the measurement value obtained from the second sensor device 29 is already representative of the electrical conductivity $s_0$ of the untreated water (corrected for deviations from a reference temperature).

In the embodiment of Fig. 4, the control device 6 uses a measurement value from the (first) sensor device 15 and a measurement value from the second sensor device 29 to determine a difference $\Delta s \equiv s_0 - s_1$ between the electrical conductivity of the untreated and the treated water. To this end, the control device converts the measurement value s(x) obtained from the (first) sensor device 15 and the measurement value $s_0$ from the second sensor device 29 into a value corresponding to the difference value $\Delta s \equiv s_0 - s_1$ as follows:

$$\Delta s = \frac{s_0 - s(x)}{1 - x} \; . \qquad\qquad (3)$$

The temporary hardness is determined by dividing this value by the factor F of which the value is determined using the linear relation between the factor F and the average $\bar{s}$ of the absolute value of the conductivity of the untreated water and the water at the exit point 19. The average value $\bar{s}$ can be readily determined as:

$$\bar{s} = \frac{\tfrac{1}{2} s(x) + s_0 \left( \tfrac{1}{2} - x \right)}{1 - x} \; . \qquad\qquad (4)$$

A variant is possible in which the (first) sensor device 15 and second sensor device 29 are calibrated by setting the blending fraction at one (i.e. 100 % bypass) and comparing the measurements from the two devices 15,29 to eliminate systematic errors. Such an operation could be carried out, for example, at the same time as the replacement of the fluid treatment device 3 and with the water from the outlet 2 flushed down the drain to avoid damage to any appliances normally connected to the outlet 2.

An even simpler variant of a system for determining a measure of hardness is illustrated in Fig. 5. Such a system could be used as a monitoring device, e.g. by diverting a small fraction of a flow of water. The system includes an inlet 34, an outlet 35 and a fluid treatment device 36. It further includes a first sensor device 37 positioned upstream of the fluid treatment device 36 and a second sensor device 38 positioned downstream of the fluid treatment device 36.

The fluid treatment device 36 is arranged, for example, to remove temporary hardness to a certain extent, e.g. completely. The sensor devices 37,38 are arranged to measure the electrical conductivity $s_0$ of the untreated water (in the case of the first sensor device 37) and the electrical conductivity $s_1$ of the treated water (in the case of the second sensor device 38). A data processing device 39 receives the measurement values $s_0, s_1$, through appropriate interfaces 40,41, determines the difference $\Delta s$, and divides this difference by a factor F. To obtain a value of the factor F, the data processing device 39 averages the measurement values $s_0, s_1$ to obtain an average value $\bar{s}$ and proceeds to calculate a value of the factor F using a linear relation $F = -a \cdot \bar{s} + b$. The result is provided as output through an interface 42 to a device (not shown) for displaying and/or processing the measure of the temporary hardness, and is more accurate than if a constant factor F had been used.

The invention is not limited to the embodiments described above, but may be varied within the scope of the accompanying claims. For example, the method may use pH values rather than electrical conductivity values. Instead of taking the average absolute conductivity value $\bar{s}$, a less accurate embodiment may use one or the average of the two measurement values obtained in the second and fourth steps 21,23 of the method illustrated in Fig. 2 or it may use only the electrical conductivity $s_0$ of the untreated water or the electrical conductivity $s_1$ of the treated water at the exit point 19, despite the fact that this would reduce the accuracy of the conversion into a value representative of the temporary hardness somewhat.

LIST OF REFERENCE NUMERALS

| | |
|---|---|
| 1 | - inlet |
| 2 | - outlet |
| 3 | - fluid treatment device |
| 4 | - variable ratio flow divider |
| 5 | - motor |
| 6 | - control device |
| 7 | - interface to motor |
| 8 | - mixing location |
| 9 | - data processing unit |
| 10 | - memory |
| 11 | - interface |
| 12 | - interface to flow meter |
| 13 | - flow meter |
| 14 | - interface to sensor device |
| 15 | - sensor device |
| 16 | - conductivity sensor |
| 17 | - temperature sensor |
| 18 | - data processor |
| 19 | - exit point |
| 20 | - step (set first blending fraction) |
| 21 | - step (obtain first conductivity value) |
| 22 | - step (set second blending value) |
| 23 | - step (obtain second conductivity value) |
| 24 | - step (determine difference value) |
| 25 | - step (divide by blending fraction differential) |
| 26 | - step (determine average) |
| 27 | - step (determine conversion factor) |
| 28 | - step (convert to temporary hardness) |
| 29 | - second sensor device |
| 30 | - second conductivity sensor |
| 31 | - second temperature sensor |
| 32 | - interface to second sensor device |
| 33 | - data processor |
| 34 | - inlet |
| 35 | - outlet |
| 36 | - fluid treatment device |

37    - first sensor device
38    - second sensor device
39    - data processing device
40    - interface to first sensor device
41    - interface to second sensor device
42    - interface for providing output

**Claims**

1.  Method of determining a measure of a concentration of components removable from fluid in the form of a liquid by a fluid treatment part of a fluid treatment device, including the use of a fluid treatment apparatus including:

    an inlet (1;34) for receiving untreated fluid;
    an outlet (2;35); and
    at least one fluid path between the inlet (1;34) and the outlet (2;35) including at least one fluid treatment part (3;36) in the form of a liquid treatment part for treating fluid to remove to at least a certain extent at least some types of components in fluid led through the fluid treatment part (3;36), the method including:

    obtaining at least two measurement values, each representative of a respective value of a parameter of the fluid depending partly on the concentration of components removable by the fluid treatment part (3;36), wherein the measurement values pertain to measurements carried out on fluid at different ratios of untreated fluid to fluid led through the at least one fluid path including a fluid treatment part (3;36);
    using the measurement values to obtain a difference value representative of a change in the value of the parameter due to treatment by the fluid treatment part (3;36); and
    converting the difference value into an output value representative of the concentration of components removable from fluid by a fluid treatment part (3;36), **characterised in that**
    the conversion is carried out in dependence on a further value of the parameter approximating absolute values of the parameter within the fluid treatment apparatus.

2.  Method according to claim 1,
    wherein the further value at least approximates absolute values of the parameter directly upstream and directly downstream of the fluid treatment part (3;36).

3.  Method according to claim 2,
    wherein the further value corresponds to an average of the values of the parameter directly upstream and downstream of the fluid treatment part (3;36).

4.  Method according to any one of the preceding claims,
    wherein converting the difference value into an output value includes dividing the difference value by a factor linearly dependent on the further value.

5.  Method according to according to any one of the preceding claims,
    wherein the measurement values are based on measurements of the electrical conductivity of the fluid.

6.  Method according to any one of the preceding claims,
    wherein the measurement values are based on measured values corrected to take account of deviations of temperature from a pre-determined reference value of the temperature.

7.  Method according to any one of the preceding claims,
    wherein the measurement values are obtained from the same sensor (15) at different points in time.

8.  Method according to claim 7,
    wherein the fluid treatment apparatus further includes:

    a branch point between the inlet (1), the at least one fluid path including at least one fluid treatment part (3) and at least one second fluid path, each second fluid path bypassing at least one of the fluid treatment parts (3), the second fluid paths conducting, in use, a blending fraction between zero and one of the fluid received through the inlet (1);
a mixing location (8), where the first and second fluid paths join; and
at least one device (4,5) for adjusting the blending fraction,
wherein the method includes obtaining the measurement values from at least one sensor (15) located downstream of the mixing location (8) and causing the at least one device (4,5) to adjust the blending fraction between the measurements carried out at different ratios of untreated fluid to fluid led through the at least one fluid path including at least one fluid treatment part (3).

9. Method according to any one of the preceding claims,
wherein the fluid treatment part (3;36) is a liquid treatment part, in particular a liquid treatment part including an ion exchange medium, more particularly a liquid treatment part including an ion exchange medium that is at least initially in a hydrogen form.

10. Method according to any one of the preceding claims, including:

obtaining a measure of an amount of fluid led through at least one fluid path including at least one fluid treatment part (3;36); and
using at least this measure and a measure of the concentration of components removable from fluid determined for the certain time period in a determination of a measure of a state of exhaustion of the fluid treatment part (3;36).

11. Method according to any one of the preceding claims,
wherein the liquid treatment part includes an ion exchange medium.

12. System for determining a measure of a concentration of components removable from fluid in the form of a liquid by a fluid treatment part (3;36) of a fluid treatment device based on measurements obtained using a fluid treatment apparatus including:

an inlet (1;34) for receiving untreated fluid;
an outlet (2;35); and
at least one fluid path between the inlet (1;34) and the outlet (2;35) including at least one fluid treatment part (3;36) in the form of a liquid treatment part for treating fluid to remove to at least a certain extent at least some types of components in fluid led through the fluid treatment part (3;36), the system including:

an interface (14;32;40,41) to at least one sensor (15;29;37,38) for obtaining at least two measurement values, each representative of a respective value of a parameter of the fluid depending partly on the concentration of components removable by the fluid treatment part (3;36),
wherein the measurement values pertain to measurements carried out on fluid at different ratios of untreated fluid to fluid led through the fluid path including the fluid treatment part (3;36),
wherein the system is configured to use the measurement values to obtain a difference value representative of a change in the value of the parameter due to treatment by the fluid treatment part (3;36); and
to convert the difference value into an output value representative of the concentration of components removable from fluid by a fluid treatment part (3;36), **characterised in that**
the system is configured to carry out the conversion in dependence on a further value of the parameter approximating absolute values of the parameter within the fluid treatment apparatus.

13. System according to claim 12, configured to carry out a method according to any one of claims 1-11.

14. System for treating a fluid in the form of a liquid, including at least:

an inlet (1;34) for receiving untreated fluid;
an outlet (2; 35) ;
at least one fluid path (1;34) between the inlet and the outlet (2;35) including a fluid treatment part (3;36) in the form of a liquid treatment part for treating fluid to remove to at least a certain extent at least some types of components in fluid led through the fluid treatment part (3;36); and
a system according to claim 12 or 13.

15. Computer program including a set of instructions capable, when incorporated into a machine-readable medium, of causing a system (6;39) having information processing capabilities to carry out a method according to any one of claims 1-11.

**Patentansprüche**

1. Verfahren zum Bestimmen eines Maßes einer Konzentration von Komponenten, die aus einem Fluid in Form einer Flüssigkeit durch einen Fluidbehandlungteil einer Fluidbehandlungsvorrichtung entfernbar sind, das die Verwendung einer Fluidbehandlungsvorrichtung umfasst, die Folgendes enthält:

einen Einlass (1; 34) zum Empfangen unbehandelten Fluids;
einen Auslass (2; 35); und
wenigstens einen Fluidweg zwischen dem Einlass (1; 34) und dem Auslass (2; 35), der wenigstens einen Fluidbehandlungteil (3; 36) in der Form eines Flüssigkeitsbehandlungsteils zum Behandeln eines Fluids, um zu mindestens einem bestimmten Ausmaß zumindest einige Typen von Komponenten in Fluid, das durch den Fluidbehandlungteil (3; 36) geführt wird, zu entfernen, enthält, wobei das Verfahren Folgendes umfasst:

ein Beziehen wenigstens zweier Messwerte, die jeweils einen jeweiligen Wert eines Parameters des Fluids, der teilweise von der Konzentration von Komponenten, die durch den Fluidbehandlungteil (3; 36) entfernbar sind, abhängt, repräsentieren,
wobei die Messwerte Messungen betreffen, die an dem Fluid bei verschiedenen Verhältnissen von unbehandeltem Fluid zu Fluid, das durch den wenigstens einen einen Fluidbehandlungteil (3; 36) aufweisenden Fluidweg hindurchgeführt wird, ausgeführt werden;
Verwenden der Messwerte, um einen Unterschiedswert zu erhalten, der eine Änderung des Werts des Parameters infolge der Behandlung durch den Fluidbehandlungteil (3; 36) repräsentiert; und
Umsetzen des Unterschiedswerts in einen Ausgabewert, der die Konzentration von Komponenten, die aus dem Fluid durch einen Fluidbehandlungteil (3; 36) entfernbar sind, repräsentiert, **dadurch gekennzeichnet, dass** die Umsetzung abhängig von einem weiteren Wert des Parameters, der absolute Werte des Parameters innerhalb der Fluidbehandlungsvorrichtung annähert, ausgeführt wird.

2. Verfahren nach Anspruch 1,
wobei der weitere Wert zumindest teilweise absolute Werte des Parameters unmittelbar zulaufseitig und unmittelbar ablaufseitig des Fluidbehandlungsteils (3; 36) annähert.

3. Verfahren nach Anspruch 2,
wobei der weitere Wert einem Mittelwert der Werte des Parameters unmittelbar zulaufseitig und ablaufseitig des Fluidbehandlungsteils (3; 36) entspricht.

4. Verfahren nach einem der vorhergehenden Ansprüche,
wobei das Umsetzen des Unterschiedwerts in einen Ausgabewert umfasst, den Unterschiedswert durch einen Faktor, der von dem weiteren Wert linear abhängt, zu teilen.

5. Verfahren nach einem der vorhergehenden Ansprüche,
wobei die Messwerte auf Messungen der elektrischen Leitfähigkeit des Fluids beruhen.

6. Verfahren nach einem der vorhergehenden Ansprüche,
wobei die Messwerte auf gemessenen Werten, die korrigiert werden, um Abweichungen der Temperatur von einem vorbestimmten Referenzwert der Temperatur zu berücksichtigen, beruhen.

7. Verfahren nach einem der vorhergehenden Ansprüche,
wobei die Messwerte von demselben Sensor (15) zu verschiedenen Zeitpunkten erhalten werden.

8. Verfahren nach Anspruch 7,
wobei die Fluidbehandlungsvorrichtung ferner Folgendes enthält:

einen Zweigpunkt zwischen dem Einlass (1), dem mindestens einen Fluidweg, der wenigstens einen Fluidbehandlungteil (3) enthält, und wenigstens einem zweiten Fluidweg, wobei jeder zweite Fluidweg wenigstens einen der Fluidbehandlungsteile (3) umgeht, wobei die zweiten Fluidwege in Betrieb einen Verschnittanteil zwischen Null und Eins des Fluids, das durch den Einlass (1) empfangen wird, leiten;

einen Mischort (8), wo sich die ersten und die zweiten Fluidwege vereinigen; und

mindestens eine Vorrichtung (4, 5) zum Anpassen des Verschnittanteil, wobei das Verfahren umfasst, die Messwerte von mindestens einem Sensor (15), der sich ablaufseitig des Mischorts (8) befindet, zu beziehen, und zu bewirken, dass die wenigstens eine Vorrichtung (4, 5) den Verschnittanteil zwischen den Messungen, die bei verschiedenen Verhältnissen von unbehandeltem Fluid zu Fluid, das durch den wenigstens einen den wenigstens einen Fluidbehandlungsteil (3) enthaltenden Fluidweg hindurchgeführt wird, ausgeführt werden, anpasst.

9. Verfahren nach einem der vorhergehenden Ansprüche,
wobei der Fluidbehandlungsteil (3; 36) einen Flüssigkeitsbehandlungsteil, insbesondere einen Flüssigkeitsbehandlungsteil, der ein Ionenaustauschmedium enthält, noch spezieller einen Flüssigkeitsbehandlungsteil, der ein Ionenaustauschmedium enthält, das sich zumindest anfangs in der Wasserstoffform befindet, enthält.

10. Verfahren nach einem der vorhergehenden Ansprüche, das Folgendes umfasst:

Erhalten eines Maßes einer Menge von Fluid, das durch mindestens einen Fluidweg, der wenigstens einen Fluidbehandlungsteil (3; 36) enthält, geführt wird; und

Verwenden zumindest dieses Maßes und eines Maßes der Konzentration von Komponenten, die aus dem Fluid entfernbar sind, das für die bestimmte Zeitperiode bestimmt wird, bei einer Bestimmung eines Maßes des Erschöpfungszustands des Fluidbehandlungsteils (3; 36).

11. Verfahren nach einem der vorhergehenden Ansprüche,
wobei der Flüssigkeitsbehandlungsteil ein Ionenaustauschmedium enthält.

12. System zum Bestimmen eines Maßes einer Konzentration von Komponenten, die aus einem Fluid in Form einer Flüssigkeit durch einen Fluidbehandlungsteil (3; 36) einer Fluidbehandlungsvorrichtung entfernbar sind, anhand von Messungen, die durch die Verwendung einer Fluidbehandlungsvorrichtung erhalten werden, das Folgendes umfasst:

einen Einlass (1; 34) zum Empfangen unbehandelten Fluids;
einen Auslass (2; 35); und
wenigstens einen Fluidweg zwischen dem Einlass (1; 34) und dem Auslass (2; 35), der wenigstens einen Fluidbehandlungsteil (3; 36) in Form eines Flüssigkeitsbehandlungsteils zum Behandeln eines Fluids, um zu mindestens einem bestimmten Ausmaß zumindest einige Typen von Komponenten in Fluid, das durch den Fluidbehandlungsteil (3; 36) geführt wird, zu entfernen, enthält, wobei das System Folgendes umfasst:

eine Schnittstelle (14; 32; 40; 41) zu wenigstens einem Sensor (15; 29; 37, 38) zum Erhalten wenigstens zweier Messwerte, die jeweils einen jeweiligen Wert eines Parameters des Fluids, der teilweise von der Konzentration von Komponenten, die durch den Fluidbehandlungsteil (3; 36) entfernbar sind, abhängt, repräsentieren,
wobei die Messwerte Messungen betreffen, die an dem Fluid bei verschiedenen Verhältnissen von unbehandeltem Fluid zu durch den wenigstens einen Fluidweg, der einen Fluidbehandlungsteil (3; 36) enthält, hindurchgeführtem Fluid ausgeführt werden;
wobei das System konfiguriert ist, die Messwerte zu verwenden, um einen Unterschiedswert zu erhalten, der eine Änderung des Werts des Parameter infolge der Behandlung durch den Fluidbehandlungsteil (3; 36) repräsentiert; und
den Unterschiedswert in einen Ausgabewert umzusetzen, der die Konzentration von Komponenten, die aus dem Fluid durch einen Fluidbehandlungsteil (3; 36) entfernbar sind, repräsentiert, **dadurch gekennzeichnet, dass**
das System konfiguriert ist, die Umsetzung abhängig von einem weiteren Wert des Parameters, der absolute Werte des Parameters innerhalb der Fluidbehandlungsvorrichtung annähert, auszuführen.

13. System nach Anspruch 12, das konfiguriert ist, ein Verfahren nach einem der Ansprüche 1-11 auszuführen.

14. System zum Behandeln eines Fluids in Form einer Flüssigkeit, das zumindest Folgendes umfasst:

einen Einlass (1; 34) zum Empfangen eines unbehandelten Fluids;
einen Auslass (2; 35);

wenigstens einen Fluidweg (1; 34) zwischen dem Einlass und dem Auslass (2; 35), der einen Fluidbehandlungsteil (3; 36) in Form eines Flüssigkeitsbehandlungsteils zum Behandeln eines Fluids, um zu mindestens einem bestimmten Ausmaß zumindest einige Typen von Komponenten in Fluid, das durch den Fluidbehandlungsteil (3; 36) geführt wird, zu entfernen, enthält; und

ein System nach Anspruch 12 oder 13.

15. Computerprogramm, das eine Gruppe von Anweisungen enthält, die dann, wenn sie in ein maschinenlesbares Medium implementiert sind, bewirken können, dass ein System (6; 39) mit Datenverarbeitungsfähigkeiten ein Verfahren nach einem der Ansprüche 1-11 ausführt.

## Revendications

1. Procédé de détermination d'une mesure d'une concentration de composants pouvant être retirés d'un fluide sous la forme d'un liquide par une partie de traitement de fluide d'un dispositif de traitement de fluide, comprenant l'utilisation d'un appareil de traitement de fluide comprenant :

une admission (1; 34) permettant de recevoir un fluide non traité ;
une évacuation (2; 35) ; et
au moins un trajet de fluide entre l'admission (1; 34) et l'évacuation (2; 35) comprenant au moins une partie de traitement de fluide (3; 36) sous la forme d'une partie de traitement de liquide permettant de traiter un fluide pour retirer au moins dans une certaine mesure au moins certains types de composants dans le fluide passant par la partie de traitement de fluide (3; 36), le procédé comprenant :

l'obtention d'au moins deux valeurs de mesure, chacune représentant une valeur respective d'un paramètre du fluide dépendant en partie de la concentration de composants pouvant être retirés par la partie de traitement de fluide (3; 36),
dans lequel les valeurs de mesure se rapportent à des mesures réalisées sur un fluide à différents rapports de fluide non traité/fluide passant par le(s) trajet(s) de fluide comprenant une partie de traitement de fluide (3; 36) ;
l'utilisation des valeurs de mesure pour obtenir une valeur de différence représentant une modification de la valeur du paramètre en raison du traitement par la partie de traitement de fluide (3; 36) ; et
la conversion de la valeur de différence en une valeur de sortie représentant la concentration de composants pouvant être retirés du fluide par une partie de traitement de fluide (3; 36), **caractérisé en ce que**
la conversion est réalisée en fonction d'une valeur supplémentaire du paramètre s'approchant de valeurs absolues du paramètre à l'intérieur de l'appareil de traitement de fluide.

2. Procédé selon la revendication 1,
dans lequel la valeur supplémentaire s'approche au moins de valeurs absolues du paramètre directement en amont et directement en aval de la partie de traitement de fluide (3; 36).

3. Procédé selon la revendication 2,
dans lequel la valeur supplémentaire correspond à une moyenne des valeurs du paramètre directement en amont et en aval de la partie de traitement de fluide (3; 36).

4. Procédé selon l'une quelconque des revendications précédentes, dans lequel la conversion de la valeur de différence en une valeur de sortie comprend la division de la valeur de différence par un facteur dépendant linéairement de la valeur supplémentaire.

5. Procédé selon l'une quelconque des revendications précédentes, dans lequel les valeurs de mesure sont basées sur des mesures de la conductivité électrique du fluide.

6. Procédé selon l'une quelconque des revendications précédentes, dans lequel les valeurs de mesure sont basées sur des valeurs mesurées corrigées pour prendre en compte des écarts de température à partir d'une valeur de référence prédéterminée de la température.

7. Procédé selon l'une quelconque des revendications précédentes, dans lequel les valeurs de mesure sont obtenues à partir du même capteur (15) à différents moments.

**8.** Procédé selon la revendication 7,
dans lequel l'appareil de traitement de fluide comprend en outre :

un point de ramification entre l'admission (1), le(s) trajet(s) de fluide comprenant au moins une partie de traitement de fluide (3) et au moins un second trajet de fluide, chaque second trajet de fluide contournant au moins l'une des parties de traitement de fluide (3), les seconds trajets de fluide conduisant, lors de l'utilisation, une fraction de mélange entre zéro et un du fluide reçu par le biais de l'admission (1) ;
un emplacement de mélange (8), où les premiers et seconds trajets de fluide se rejoignent ; et
au moins un dispositif (4, 5) permettant d'ajuster la fraction de mélange, dans lequel le procédé comprend l'obtention des valeurs de mesure à partir d'au moins un capteur (15) situé en aval de l'emplacement de mélange (8) et amenant le(s) dispositif(s) (4, 5) à ajuster la fraction de mélange entre les mesures réalisées à différents rapports de fluide non traité/fluide passant par le(s) trajet(s) de fluide comprenant au moins une partie de traitement de fluide (3).

**9.** Procédé selon l'une quelconque des revendications précédentes,
dans lequel la partie de traitement de fluide (3; 36) est une partie de traitement de liquide, en particulier une partie de traitement de liquide comprenant un milieu d'échange d'ions, plus particulièrement une partie de traitement de liquide comprenant un milieu d'échange d'ions qui est au moins initialement sous forme d'ion d'hydrogène.

**10.** Procédé selon l'une quelconque des revendications précédentes, comprenant :

l'obtention d'une mesure d'une quantité de fluide passant par au moins un trajet de fluide comprenant au moins une partie de traitement de fluide (3; 36) ; et
l'utilisation d'au moins cette mesure et d'une mesure de la concentration de composants pouvant être retirés d'un fluide déterminé pendant la période déterminée dans une détermination d'une mesure d'un état d'épuisement de la partie de traitement de fluide (3; 36).

**11.** Procédé selon l'une quelconque des revendications précédentes,
dans lequel la partie de traitement de liquide comprend un milieu d'échange d'ions.

**12.** Système permettant de déterminer une mesure d'une concentration de composants pouvant être retirés d'un fluide sous la forme d'un liquide par une partie de traitement de fluide (3; 36) d'un dispositif de traitement de fluide sur la base de mesures obtenues au moyen d'un appareil de traitement de fluide comprenant :

une admission (1; 34) permettant de recevoir un fluide non traité ;
une évacuation (2; 35) ; et
au moins un trajet de fluide entre l'admission (1; 34) et l'évacuation (2; 35) comprenant au moins une partie de traitement de fluide (3; 36) sous la forme d'une partie de traitement de liquide permettant de traiter un fluide pour retirer au moins dans une certaine mesure au moins certains types de composants dans un fluide passant par la partie de traitement de fluide (3; 36), le système comprenant :

une interface (14; 32; 40; 41) avec au moins un capteur (15; 29; 37; 38) permettant d'obtenir au moins deux valeurs de mesure, chacune représentant une valeur respective d'un paramètre du fluide dépendant en partie de la concentration de composants pouvant être retirés par la partie de traitement de fluide (3; 36), dans lequel les valeurs de mesure se rapportent à des mesures réalisées sur un fluide à différents rapports de fluide non traité/fluide passant par le trajet de fluide comprenant la partie de traitement de fluide (3; 36), dans lequel le système est configuré pour utiliser les valeurs de mesure pour obtenir une valeur de différence représentant une modification de la valeur du paramètre en raison d'un traitement par la partie de traitement de fluide (3; 36) ; et
pour convertir la valeur de différence en une valeur de sortie représentant la concentration de composants pouvant être retirés d'un fluide par une partie de traitement de fluide (3; 36), **caractérisé en ce que** le système est configuré pour réaliser la conversion en fonction d'une valeur supplémentaire du paramètre s'approchant de valeurs absolues du paramètre à l'intérieur de l'appareil de traitement de fluide.

**13.** Système selon la revendication 12, configuré pour mettre en oeuvre un procédé selon l'une quelconque des revendications 1 à 11.

**14.** Système de traitement d'un fluide sous la forme d'un liquide, comprenant au moins :

une admission (1; 34) permettant de recevoir un fluide non traité ;

une évacuation (2; 35) ;

au moins un trajet de fluide (1; 34) entre l'admission et l'évacuation (2; 35) comprenant une partie de traitement de fluide (3; 36) sous la forme d'une partie de traitement de liquide permettant de traiter un fluide pour retirer au moins dans une certaine mesure au moins certains types de composants dans un fluide passant par la partie de traitement de fluide (3; 36) ; et

un système selon la revendication 12 ou 13.

15. Programme informatique comprenant un ensemble d'instructions capable, lorsqu'il est incorporé dans un support lisible par machine, d'amener un système (6; 39) présentant des capacités de traitement d'informations à mettre en oeuvre un procédé selon l'une quelconque des revendications 1 à 11.

Fig. 1

Set 1st blending fraction value — 20

Obtain 1st conductivity value — 21

Set 2nd bypass fraction value — 22

Obtain 2nd conductivity value — 23

Determine difference value — 24

Divide by blending fraction differential — 25

Determine average of conductivity values upstream and downstream of filter — 26

Determine conversion factor — 27

Convert to temporary hardness — 28

Fig. 2

Fig. 3

Fig. 5

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102010001373 A1
- EP 2169393 A1